# EUROPEAN PATENT SPECIFICATION

(11) **EP 4 595 900 B1**
(45) Date of publication and mention of the grant of the patent: **04.11.2026**
(21) Application number: 25154937.4
(22) Date of filing: 30.01.2025
(51) Int. Cl.: A61B 8/08, A61B 1/00, A61B 1/313, A61B 8/12, A61B 17/34, A61B 34/10, A61B 90/00

(54) **PUNCTURE SUPPORT APPARATUS, OPERATION METHOD THEREOF, AND NON-TRANSITORY COMPUTER READABLE MEDIUM**
PUNKTIONSUNTERSTÜTZUNGSVORRICHTUNG, BETRIEBSVERFAHREN DAFÜR UND NICHTTRANSITORISCHES COMPUTERLESBARES MEDIUM
APPAREIL DE SUPPORT DE PONCTION, SON PROCÉDÉ DE FONCTIONNEMENT ET SUPPORT NON TRANSITOIRE LISIBLE PAR ORDINATEUR

(30) Priority: 31.01.2024 JP 2024012552
(43) Date of publication of application: 06.08.2025
(73) Proprietor: FUJIFILM Corporation, Tokyo 106-8620 (JP)
(72) Inventor: SASUGA, Saeko, Tokyo, 106-8620 (JP); DOKKO, Shuichi, Chiba, 277-0804 (JP)
(74) Representative: Dehns Germany Partnerschaft mbB

(56) References cited:
- US-A1- 2019 223 958
- US-A1- 2021 307 838

## Description

### BACKGROUND OF THE INVENTION

### 1. Field of the Invention

The present invention relates to a puncture support apparatus that supports an operation of a puncture needle used in laparoscopic surgery or the like, an operation method thereof, and a non-transitory computer readable medium.

### 2. Description of the Related Art

In the related art, although a laparotomy has been widely performed, laparoscopic surgery and robot surgery as less invasive surgery have been widely used. These surgical methods have an advantage that the amount of incision in a body of a patient is small because a small port hole is provided in the body and a surgical tool is inserted through the port hole.

In addition, as the surgical tool used in the laparoscopic surgery, there is an ultrasound probe for detecting a target region distributed in a depth direction in the body, such as a lesion portion. A distal end of the ultrasound probe used in the laparoscopic surgery is provided with, for example, a puncture hole or groove, and a puncture needle is passed through the puncture hole or groove of the ultrasound probe, so that it is possible to puncture an organ at an any angle (refer to, for example, JP2022-80023A). The puncture needle is used, for example, to inject indocyanine green (ICG) or indigo carmine into a portal vein blood vessel in order to determine a resection site of liver cancer surgery.

Note that, in US8688196B, a magnetic position sensor incorporated in a distal end portion of a puncture needle is detected using a magnetic field generation apparatus, and a relationship between the position of the puncture needle and the position of the ultrasound probe is detected, and the position of the puncture needle and the direction in which the puncture needle moves are graphically displayed on an ultrasound tomogram.

Document US 2021/307838 A1 discloses a surgical navigation method.

Document US 2019/223958 A1 discloses a medical image guidance.

### SUMMARY OF THE INVENTION

In the laparoscopic surgery, as in the above-described advantage, the amount of incision can be reduced, but it is difficult to ascertain a positional relationship between a plurality of surgical tools from a camera image that observes the inside of the body, as compared with the laparotomy. In particular, in a case where a puncture needle is used to specify a surgical site of the liver as described above, it is difficult to appropriately insert the puncture needle only into the target blood vessel from among the blood vessels spread in the liver. Note that, in Masahiro
Hasegawa, et al., "Accuracy of augmented reality with computed tomography-based navigation in total hip arthroplasty", [online], [November 6, 2023], Internet, <URL: https://www.ncbi.nlm.nih.gov/pmc/articles/PMC10481587/>, although there is a description regarding surgical navigation for identifying a relationship between the position of the pelvis and the position of the surgical tool with an AR marker, there is no description or suggestion regarding identification of the positional relationship between the puncture needle and the surgical tool such as another ultrasound probe.

An object of an exemplary embodiment of the invention is to provide a puncture support apparatus, an operation method thereof, and a non-transitory computer readable medium which can ascertain a positional relationship between a plurality of surgical tools in a body, such as an ultrasound probe and a puncture needle, and support an operation of a puncture needle.

The present invention relates to a puncture support apparatus, an operation method thereof, and a non-transitory computer readable medium with the features of the independent claims. Further aspects are defined in the dependent claims.

### BRIEF DESCRIPTION OF THE DRAWINGS

Fig. 1 is a schematic diagram of a laparoscopic system.
Fig. 2 is an image diagram of an ultrasound image.
Fig. 3 is an explanatory diagram illustrating a first pattern of an acquisition pattern of distal end coordinates of an ultrasound probe and distal end coordinates of a puncture needle.
Fig. 4 is an explanatory diagram illustrating a second pattern of an acquisition pattern of distal end coordinates of an ultrasound probe and distal end coordinates of a puncture needle.
Fig. 5 is an explanatory diagram illustrating a third pattern of an acquisition pattern of distal end coordinates of an ultrasound probe and distal end coordinates of a puncture needle.
Fig. 6 is an explanatory diagram illustrating a fourth pattern of an acquisition pattern of distal end coordinates of an ultrasound probe and distal end coordinates of a puncture needle.
Fig. 7 is an explanatory diagram in a case where an extension line of a puncture needle overlaps a cross section guide in a first pattern of a generation pattern and a display pattern of support information.
Fig. 8 is an explanatory diagram in a case where an extension line of a puncture needle is on a front side of a cross section guide in a first pattern of a generation pattern and a display pattern of support information.
Fig. 9 is an explanatory diagram illustrating a second pattern of a generation pattern and a display pattern of support information.
Fig. 10 is an explanatory diagram illustrating a third pattern of a generation pattern and a display pattern of support information.
Fig. 11 is an explanatory diagram in a case where an extension line of a puncture needle does not overlap a puncture point in a fourth pattern of a generation pattern and a display pattern of support information.
Fig. 12 is an explanatory diagram in a case where an extension line of a puncture needle overlaps a puncture point in a fourth pattern of a generation pattern and a display pattern of support information.
Fig. 13 is an explanatory diagram illustrating a fifth pattern of a generation pattern and a display pattern of support information.
Fig. 14 is a flowchart illustrating the series of flows regarding support of an operation of a puncture needle.
Fig. 15 is an explanatory diagram in a case where an extension line of a puncture needle is on a front side of an ultrasound probe in a first pattern of a generation pattern and a display pattern of support information.
Fig. 16 is an explanatory diagram in a case where an extension line of a puncture needle overlaps an ultrasound probe in a first pattern of a generation pattern and a display pattern of support information.
Fig. 17 is an explanatory diagram in a case where an extension line of a puncture needle is on a front side of a cross section of an ultrasound image in a first pattern of a generation pattern and a display pattern of support information.
Fig. 18 is an explanatory diagram in a case where an extension line of a puncture needle overlaps a cross section of an ultrasound image in a first pattern of a generation pattern and a display pattern of support information.

### DESCRIPTION OF THE PREFERRED EMBODIMENTS

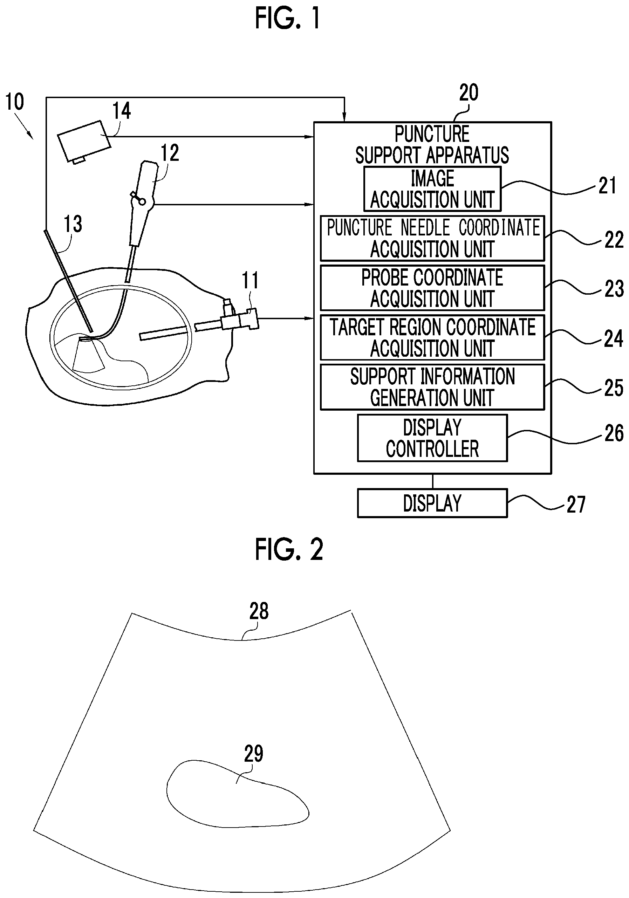

As illustrated in Fig. 1, a laparoscopic system 10 observes the inside of the body with a rigid endoscope 11, and detects a target region in a depth direction in the body, such as a lesion portion, using an ultrasound probe 12. In the laparoscopic system 10, a puncture needle 13 punctures a target region in the body at any angle. The puncture needle 13 is used, for example, to inject indocyanine green (ICG) or indigo carmine into a portal vein blood vessel in order to determine a resection site of liver cancer surgery. A camera 14 images the rigid endoscope 11, the ultrasound probe 12, or the puncture needle 13 used during laparoscopic surgery.

In the laparoscopic system 10, a puncture support apparatus 20 for supporting the insertion of the puncture needle 13 into the body is provided. A rigid endoscope image captured by the rigid endoscope 11 is transmitted to the puncture support apparatus 20 in a wired or wireless manner. An ultrasound image obtained by the ultrasound probe 12 is transmitted to the puncture support apparatus 20 in a wired or wireless manner. A camera image captured by the camera 14 is transmitted to the puncture support apparatus 20 in a wired or wireless manner. Note that the camera 14 may be a web camera or the like.

The puncture support apparatus 20 includes an image acquisition unit 21, a puncture needle coordinate acquisition unit 22, a probe coordinate acquisition unit 23, a target region coordinate acquisition unit 24, a support information generation unit 25, a display controller 26, and a display 27. The puncture support apparatus 20 is provided with a program memory (not illustrated) that stores a specific program. A controller (not illustrated) constituting a processor executes a specific program to realize functions of the image acquisition unit 21, the puncture needle coordinate acquisition unit 22, the probe coordinate acquisition unit 23, the target region coordinate acquisition unit 24, the support information generation unit 25, and the display controller 26.

The image acquisition unit 21 acquires a first image acquired by an imaging apparatus that images the puncture needle 13 or the ultrasound probe 12. The puncture needle coordinate acquisition unit 22 acquires the distal end coordinates of the puncture needle 13 on the basis of the first image. Since there are a plurality of acquisition patterns of the distal end coordinates of the puncture needle 13, the details will be described later. The probe coordinate acquisition unit 23 acquires the distal end coordinates of the ultrasound probe 12 on the basis of the first image. Since there are a plurality of acquisition patterns of the distal end coordinates of the ultrasound probe 12, the details will be described later.

The target region coordinate acquisition unit 24 acquires coordinates of a target region in the ultrasound image acquired by the ultrasound probe 12. As illustrated in Fig. 2, the target region coordinate acquisition unit 24 acquires an image of a target region 29 such as a lesion portion, by performing target region detection processing on an ultrasound image 28. The support information generation unit 25 generates support information for supporting a puncture operation of the puncture needle 13, by using at least any one of the distal end coordinates of the puncture needle 13, the distal end coordinates of the ultrasound probe 12, or the coordinates of the target region. The display controller 26 displays the support information on the display by superimposing the support information on the first image. Since there are a plurality of generation patterns and display patterns of the support information, the details will be described later.

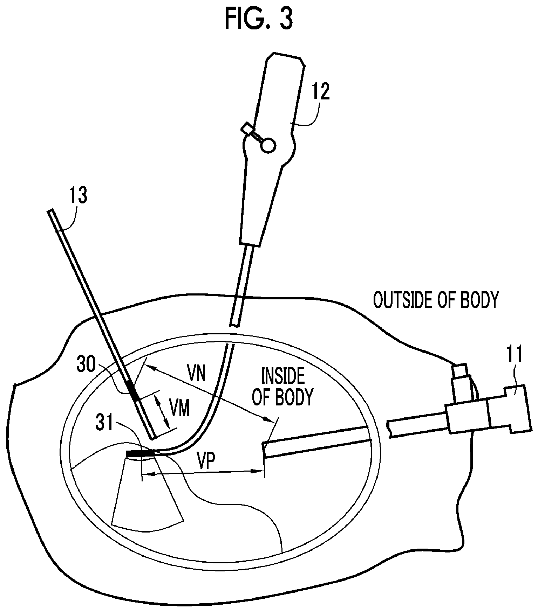

Regarding the acquisition pattern of the distal end coordinates of the ultrasound probe 12 and the distal end coordinates of the puncture needle 13, the following four patterns will be described. The first pattern is a pattern in which the distal end coordinates of the ultrasound probe 12 and the distal end coordinates of the puncture needle 13 are acquired from the image obtained in the body. As illustrated in Fig. 3, the puncture needle coordinate acquisition unit 22 acquires the distal end coordinates of the puncture needle 13 on the basis of the rigid endoscope image which is the first image. In order to acquire the distal end coordinates of the puncture needle 13, an intracorporeal puncture needle marker 30 is attached to a portion of the puncture needle 13, which is inserted into the body. The intracorporeal puncture needle marker 30 is provided at a portion separated from the distal end of the puncture needle 13 by a certain distance in the body. Therefore, the puncture needle coordinate acquisition unit 22 calculates the distal end coordinates of the puncture needle 13 from a vector VM between the intracorporeal puncture needle marker 30 and the distal end of the puncture needle 13, which is known, and a vector VN between the intracorporeal puncture needle marker 30 and the rigid endoscope 11, which is detected from the rigid endoscope image. Note that the intracorporeal puncture needle marker 30 is preferably an AR marker.

In addition, the probe coordinate acquisition unit 23 acquires the distal end coordinates of the ultrasound probe 12 on the basis of the rigid endoscope image which is the first image. In order to acquire the distal end coordinates of the ultrasound probe 12, an intracorporeal probe marker 31 is attached to a portion of the ultrasound probe 12 inserted into the body. The intracorporeal probe marker 31 is provided at the distal end of the ultrasound probe 12. Therefore, the distal end coordinates of the ultrasound probe 12 are calculated from a vector VP between the intracorporeal probe marker 31 and the rigid endoscope 11, which is detected from the rigid endoscope image. Note that the intracorporeal probe marker 31 is also preferably an AR marker.

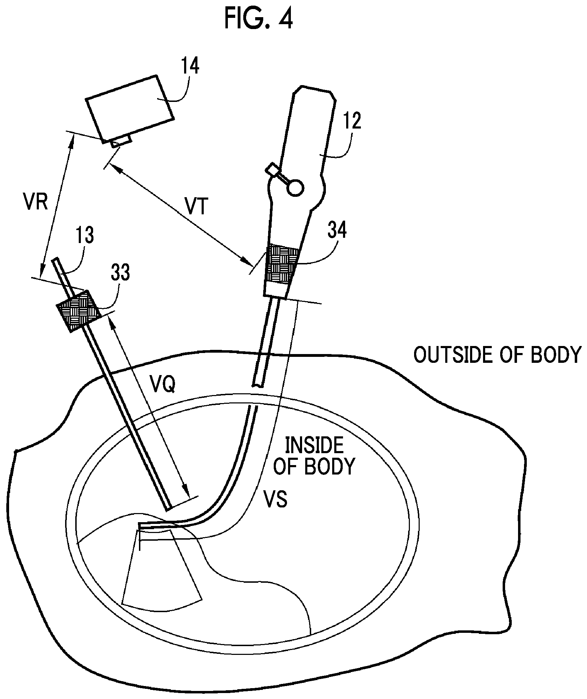

The second pattern is a pattern in which the distal end coordinates of the ultrasound probe 12 and the distal end coordinates of the puncture needle 13 are acquired from the image obtained outside the body. As illustrated in Fig. 4, the puncture needle coordinate acquisition unit 22 acquires the distal end coordinates of the puncture needle 13 on the basis of the camera image which is the first image. In order to acquire the distal end coordinates of the puncture needle 13, an extracorporeal puncture needle marker 33 is attached to a portion of the puncture needle 13 positioned outside the body. The extracorporeal puncture needle marker 33 is provided at a portion separated from the distal end of the puncture needle 13 by a certain distance outside the body. Therefore, the puncture needle coordinate acquisition unit 22 calculates the distal end coordinates of the puncture needle 13 from a vector VQ between the extracorporeal puncture needle marker 33 and the distal end of the puncture needle 13, which is known, and a vector VR between the extracorporeal puncture needle marker 33 and the camera 14, which is detected from the camera image. Note that the extracorporeal puncture needle marker 33 is preferably an AR marker.

In addition, the probe coordinate acquisition unit 23 acquires the distal end coordinates of the ultrasound probe 12 on the basis of the camera image which is the first image. In order to acquire the distal end coordinates of the ultrasound probe 12, an extracorporeal probe marker 34 is attached to a portion of the ultrasound probe 12 positioned outside the body. The extracorporeal probe marker 34 is provided at a specific portion of the ultrasound probe 12 outside the body. Therefore, the distal end coordinates of the ultrasound probe 12 are calculated from a vector VS between the extracorporeal probe marker 34 and the distal end of the ultrasound probe 12, which is known, and a vector VT between the extracorporeal probe marker 34 and the camera 14, which is detected from the camera image. Note that the extracorporeal probe marker 34 is also preferably an AR marker.

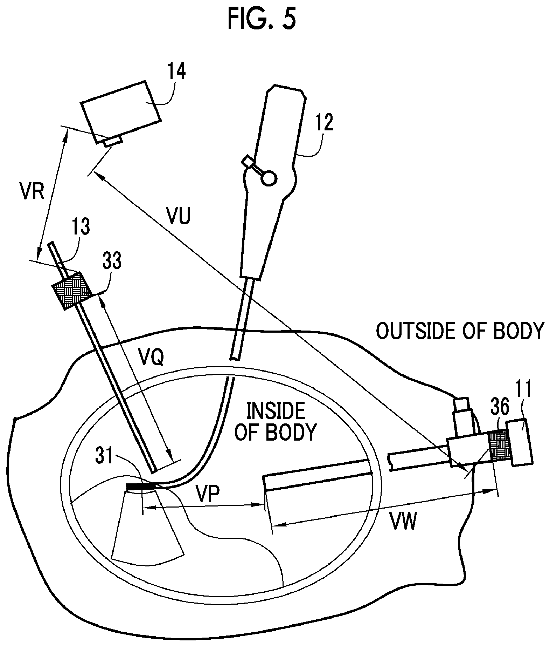

The third pattern is a pattern in which the distal end coordinates of the ultrasound probe 12 and the distal end coordinates of the puncture needle 13 are acquired from the images obtained inside and outside the body, and is different from the fourth pattern. As illustrated in Fig. 5, the puncture needle coordinate acquisition unit 22 calculates the distal end coordinates of the puncture needle 13 from the camera image which is a second image. A calculation method of the distal end coordinates of the puncture needle 13 is the same as that of the second pattern. In addition, it is preferable that the probe coordinate acquisition unit 23 calculates the distal end coordinates of the ultrasound probe 12 from the rigid endoscope image which is the first image. A calculation method of the distal end coordinates of the ultrasound probe 12 is the same as that of the first pattern. Note that, in a case where the distal end coordinates of the rigid endoscope 11 are acquired by the camera 14, it is preferable that an extracorporeal rigid endoscope marker 36 is attached to a portion of the rigid endoscope 11 positioned outside the body. In this case, the distal end coordinates of the rigid endoscope 11 are calculated on the basis of a vector VU between the camera 14 and the extracorporeal rigid endoscope marker 36, which is obtained from the camera image and a vector VW between the extracorporeal rigid endoscope marker 36 and the distal end of the rigid endoscope 11, which is known. The extracorporeal rigid endoscope marker 36 is also preferably an AR marker.

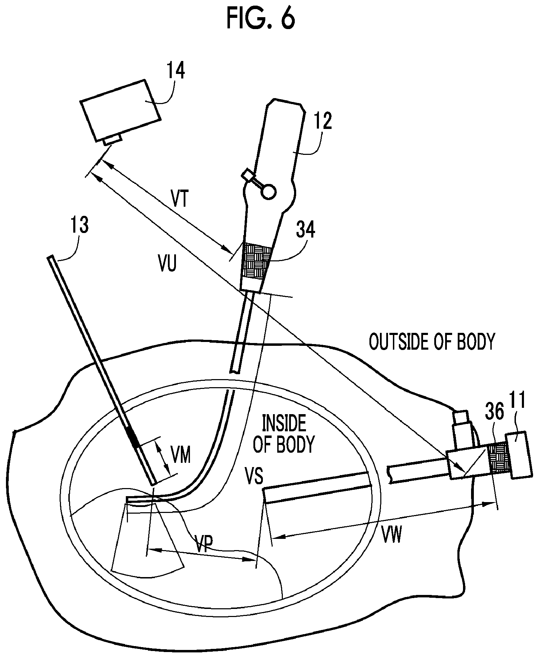

The fourth pattern is a pattern in which the distal end coordinates of the ultrasound probe 12 and the distal end coordinates of the puncture needle 13 are acquired from the images obtained inside and outside the body, and is different from the third pattern. As illustrated in Fig. 6, the puncture needle coordinate acquisition unit 22 calculates the distal end coordinates of the puncture needle 13 from the rigid endoscope image which is the first image. A calculation method of the distal end coordinates of the puncture needle 13 is the same as that of the first pattern. In addition, the probe coordinate acquisition unit 23 calculates the distal end coordinates of the ultrasound probe 12 from the camera image which is the second image. A calculation method of the distal end coordinates of the ultrasound probe 12 is the same as that of the second pattern. Note that, in a case where the distal end coordinates of the rigid endoscope 11 are acquired by the camera 14, as described above, it is preferable that the extracorporeal rigid endoscope marker 36 is attached to a portion of the rigid endoscope 11 positioned outside the body. In this case, the distal end coordinates of the rigid endoscope 11 are calculated on the basis of the vector VU between the camera 14 and the extracorporeal rigid endoscope marker 36, which is obtained from the camera image and the vector VW between the extracorporeal rigid endoscope marker 36 and the distal end of the rigid endoscope 11, which is known. The extracorporeal rigid endoscope marker 36 is also preferably an AR marker.

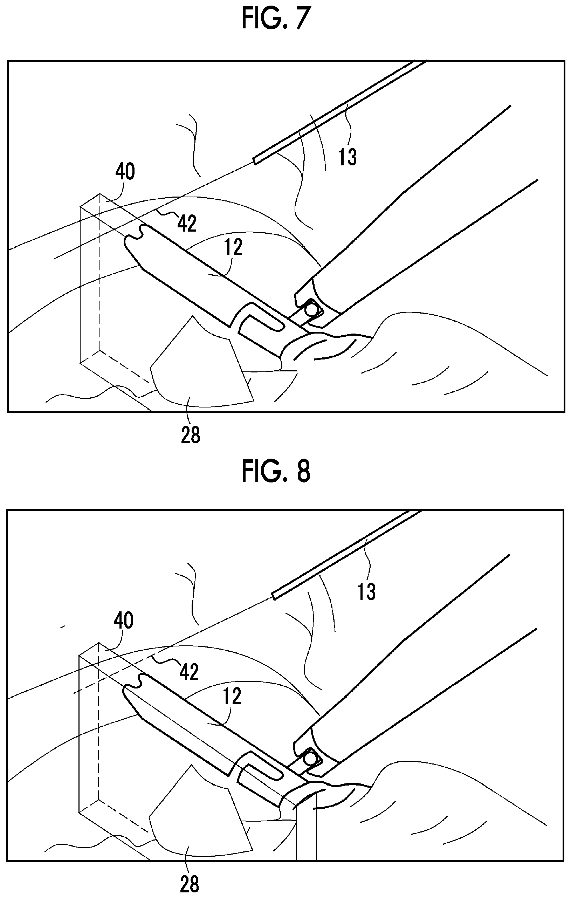

Regarding the generation pattern and the display pattern of the support information, the following five patterns will be described. In the first pattern, as illustrated in Figs. 7 and 8, the support information generation unit 25 generates a cross section guide 40 that displays the position of an ultrasound cross section, as the support information, on the basis of the distal end coordinates of the ultrasound probe 12. The display controller 26 displays the ultrasound image 28 below the ultrasound probe 12 in a superimposed manner on the rigid endoscope image, and displays the cross section guide 40 in a superimposed manner on the rigid endoscope image. The cross section guide 40 is displayed in a superimposed manner in accordance with the shape of the distal end portion of the ultrasound probe 12, and is displayed extending slightly from the distal end portion of the ultrasound probe 12. In addition, the support information generation unit 25 generates an extension line extending from the distal end of the puncture needle, as the support information, on the basis of the distal end coordinates of the puncture needle. The display controller 26 displays an extension line 42 in a superimposed manner on the rigid endoscope image.

The display controller 26 makes the display aspect of the support information different between a case where the support information is on the front side of at least any one of the cross section guide 40, the ultrasound probe 12, or the cross section of the ultrasound image 28 and a case where the support information overlaps or is on the back side of at least any one of the cross section guide 40, the ultrasound probe 12, or the cross section of the ultrasound image 28. Specifically, the display controller 26 makes the display aspect of the extension line 42 different between a case where the extension line 42 is on the front side of at least any one of the cross section guide 40, the ultrasound probe 12, or the cross section of the ultrasound image 28 and a case where the extension line 42 overlaps at least any one of the cross section guide 40, the ultrasound probe 12, or the cross section of the ultrasound image 28 or is on the back side of at least any one of the cross section guide 40, the ultrasound probe 12, or the cross section of the ultrasound image 28. As a pattern for causing the display aspect of the extension line 42 to differ, for example, a part of the extension line 42 is made different by being displayed or not displayed. In this manner, the sense of depth can be realized by displaying or not displaying the support information such as the extension line 42.

In a case where the cross section guide 40 and the extension line 42 are generated, the display controller 26 makes the display aspect of the extension line 42 different between a case where the extension line 42 is on the front side of the cross section guide 40 and a case where the extension line 42 overlaps the cross section guide 40 or is on the back side of the cross section guide 40. Specifically, in a case where the extension line 42 overlaps the cross section guide 40 or is on the back side of the cross section guide 40, the display controller 26 does not display the portion of the extension line 42, which overlaps the cross section guide 40 or is on the back side of the cross section guide 40 (displays the portion by a dotted line in Fig. 8), and in a case where the extension line 42 is on the front side of the cross section guide 40, the display controller 26 displays the extension line (displays the extension line by a solid line in Fig. 7). As a result, the sense of depth can be given to the display aspect of the extension line. Since the entire extension line 42 is displayed in Fig. 7, it can be seen that the extension line 42 is on the front side of the cross section guide 40 without being superimposed on the cross section guide 40. Since a part of the extension line is not displayed in Fig. 8, it can be seen that the non-display portion overlaps the cross section guide 40. Note that the color of the extension line 42 may be made different between a case where the extension line 42 is on the front side of the cross section guide 40 and a case where the extension line 42 overlaps the cross section guide 40 or is on the back side of the cross section guide 40 (for example, the color of the extension line 42 is blue in a case where the extension line 42 overlaps the cross section guide 40 and the color of the extension line 42 is red in a case where the extension line 42 does not overlap the cross section guide 40).

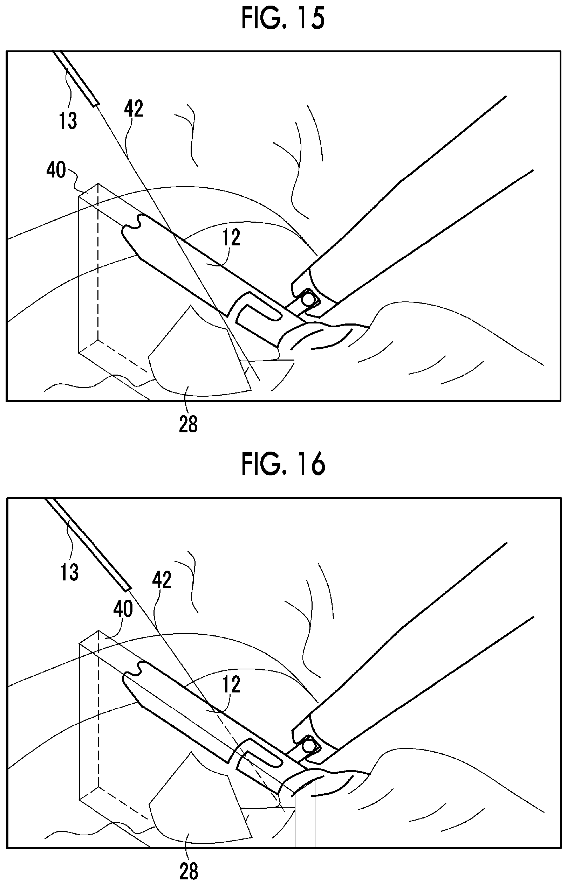

Note that the display controller 26 makes the display aspect of the extension line 42 different between a case where the extension line 42 is on the front side of the ultrasound probe 12 and a case where the extension line 42 overlaps the ultrasound probe 12 or is on the back side of the ultrasound probe 12. Specifically, as illustrated in Fig. 15, in a case where the extension line 42 is on the front side of the ultrasound probe 12, the display controller 26 displays the extension line (displays the extension line by a solid line in Fig. 15), and as illustrated in Fig. 16, in a case where the extension line 42 overlaps the ultrasound probe 12 or is on the back side of the ultrasound probe 12, the display controller 26 does not display the portion of the extension line 42, which overlaps the ultrasound probe 12 or is on the back side of the ultrasound probe 12 (displays the portion by a dotted line in Fig. 16).

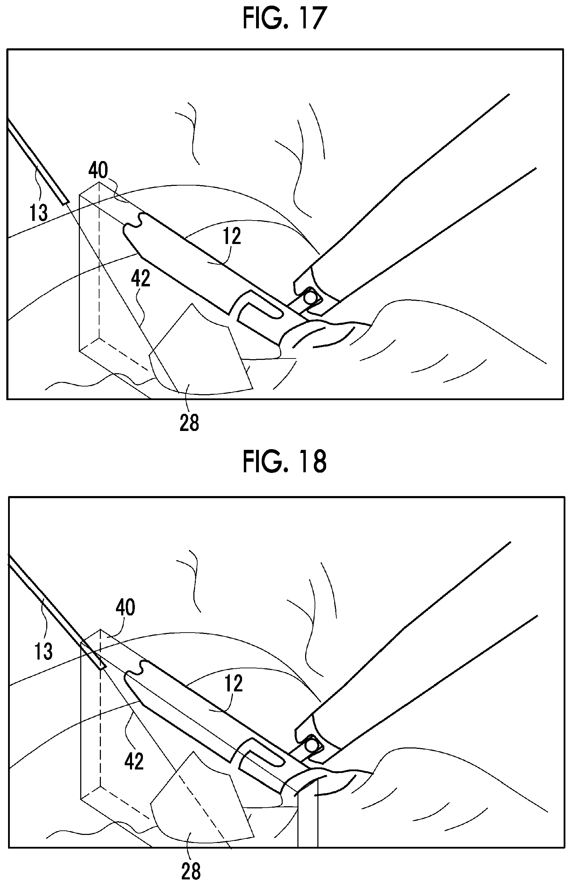

In addition, the display controller 26 makes the display aspect of the extension line 42 different between a case where the extension line 42 is on the front side of the cross section of the ultrasound image 28 and a case where the extension line 42 overlaps the cross section of the ultrasound image 28 or is on the back side of the cross section of the ultrasound image 28. Specifically, as illustrated in Fig. 17, in a case where the extension line 42 is on the front side of the cross section of the ultrasound image 28, the display controller 26 displays the extension line (displays the extension line by a solid line in Fig. 17), and as illustrated in Fig. 18, in a case where the extension line 42 overlaps the cross section of the ultrasound image 28 or is on the back side of the cross section of the ultrasound image 28, the display controller 26 does not display the portion of the extension line 42, which overlaps the cross section of the ultrasound image 28 or is on the back side of the cross section of the ultrasound image 28 (displays the portion by a dotted line in Fig. 18).

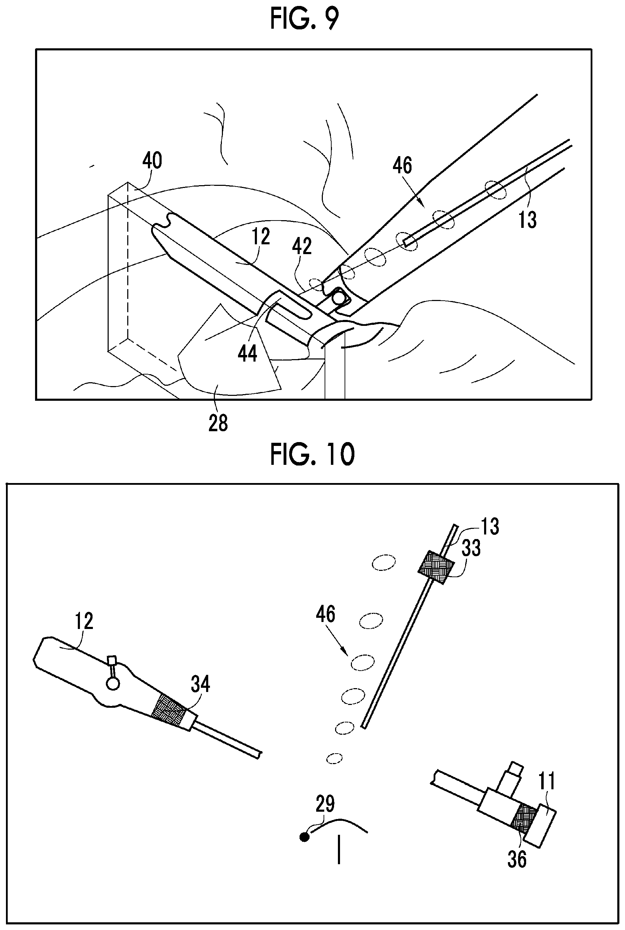

In the second pattern, as illustrated in Fig. 9, the support information generation unit 25 generates a puncture needle guide 46 for guiding the puncture needle 13 in accordance with the position of a needle insertion hole 44 of the ultrasound probe, as the support information, on the basis of the distal end coordinates of the ultrasound probe 12. The display controller 26 displays the ultrasound image below the ultrasound probe 12 in a superimposed manner on the rigid endoscope image (first image), and displays the puncture needle guide 46 in a superimposed manner on the rigid endoscope image (first image). The puncture needle guide 46 is formed of a plurality of ellipses, and the ellipses are provided at regular intervals toward the needle insertion hole 44. A user can reliably reach the needle insertion hole 44 by operating the puncture needle 13 to pass through the ellipses.

In the third pattern, as illustrated in Fig. 10, the image of the puncture needle guide 46 to be displayed in a superimposed manner in the second pattern is displayed in a superimposed manner on the camera image (first image) instead of the rigid endoscope image. As a result, it is possible to ascertain how much the current position of the puncture needle 13 is deviated from the target insertion position in the outside of the body. Note that, in the camera image, in order to display the coordinates of the target region 29, it is preferable to calculate the coordinates of the target region 29 using the ultrasound image even in the third pattern.

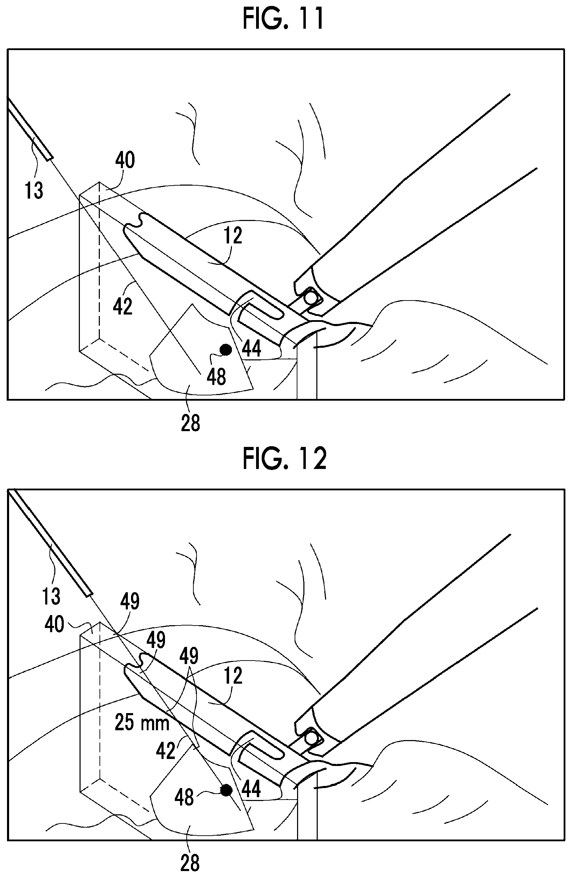

In the fourth pattern, as illustrated in Fig. 11, the support information generation unit 25 generates a puncture point 48, which is the target insertion position of the puncture needle 13, as the support information, on the basis of the coordinates of the target region. The display controller 26 displays the ultrasound image below the ultrasound probe 12 in a superimposed manner on the rigid endoscope image, and displays the puncture point 48 in the ultrasound image in a specific color and a specific shape (in Fig. 11, "O"). In addition, since the extension line 42 of the puncture needle 13 is also displayed in a superimposed manner, the positional relationship between the puncture needle 13 and the puncture point 48 can be ascertained.

In addition, in a case where the support information generation unit 25 generates the puncture point 48 and the extension line 42 of the puncture needle 13, as illustrated in Fig. 12, in a case where the extension line 42 overlaps the puncture point 48, the support information generation unit 25 calculates a distance from the distal end of the puncture needle 13 to the target region, as the support information, on the basis of the distal end coordinates of the puncture needle 13. The calculated distance is displayed on the display 27 by the display controller 26. In Fig. 12, "25 mm" is displayed as the distance, and a gradation 49 is provided at an interval of 5 mm.

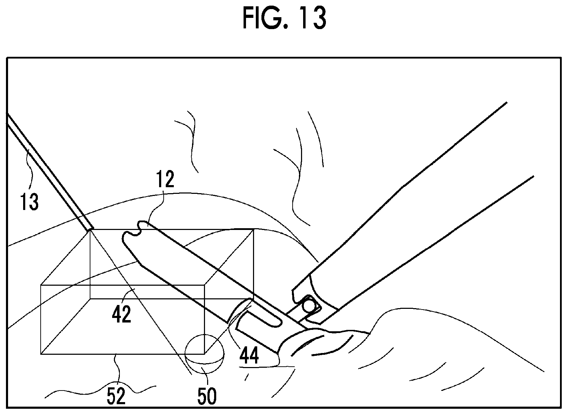

In the fifth pattern, the support information generation unit 25 generates a three-dimensional model of the target region, as the support information, on the basis of the ultrasound image 28 and the coordinates of the target region 29. As illustrated in Fig. 13, the display controller 26 displays, as the three-dimensional model of the target region 29, a shape model 50 representing the target region 29 on the display 27. In addition, the support information generation unit 25 generates a three-dimensional relationship from the puncture needle 13 to the target region 29, as the support information, on the basis of the distal end coordinates of the puncture needle 13 and the coordinates of the target region 29. As illustrated in Fig. 13, the display controller 26 displays a lattice-shaped rectangular parallelepiped 52 as the three-dimensional relationship. Since the three-dimensional relationship such as the distance and the direction between the puncture needle 13 and the target region 29 can be ascertained by the lattice-shaped rectangular parallelepiped 52, the user can easily operate the puncture needle 13.

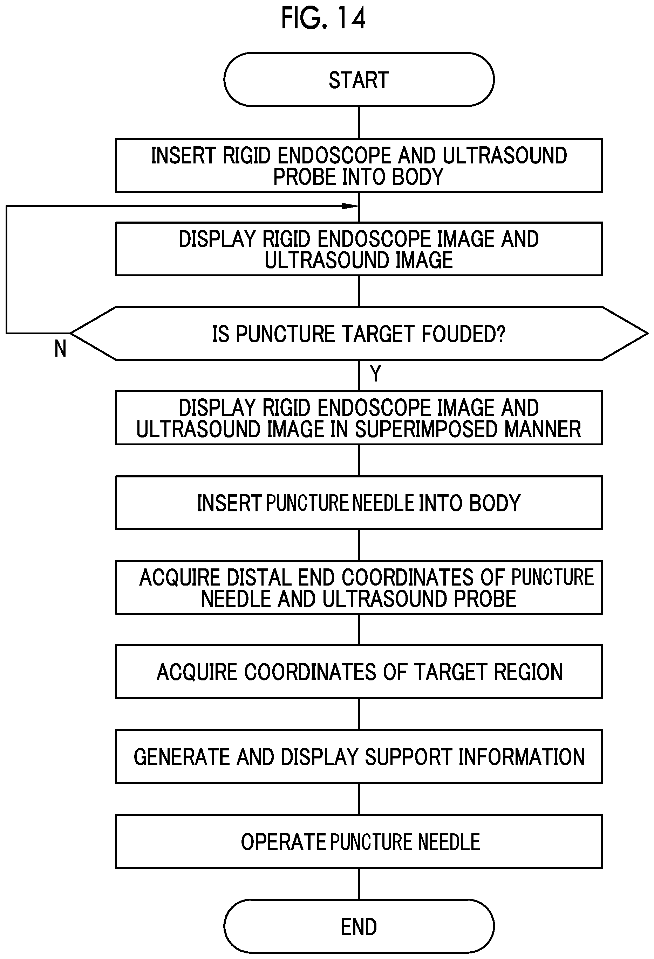

Next, the series of flows for supporting the operation of the puncture needle 13 in a case of performing surgery on the liver will be described with reference to the flowchart of Fig. 14. First, by using a trocar or the like, the rigid endoscope 11 and the ultrasound probe 12 are inserted into the body from the abdominal cavity. The ultrasound probe 12 is provided with any one of the intracorporeal probe marker 31 or the extracorporeal probe marker 34. The ultrasound image obtained by the ultrasound probe 12 and the rigid endoscope image obtained by the rigid endoscope 11 are displayed separately on the display 27. The user searches for a puncture target by placing the ultrasound probe 12 against the liver while observing the display 27. In a case where the puncture target is found by the search, a user interface (not illustrated) connected to the puncture support apparatus 20 is operated to switch to a superimposed display mode in which an ultrasound image is displayed on a rigid endoscope image in a superimposed manner. As a result, the image in which the ultrasound image is displayed in a superimposed manner on a puncture target portion in the rigid endoscope image is displayed on the display 27.

Next, the user inserts the puncture needle 13 into the body while observing the display 27. The puncture needle 13 is provided with any one of the intracorporeal puncture needle marker 30 or the extracorporeal puncture needle marker 33. The puncture needle coordinate acquisition unit 22 acquires the distal end coordinates of the puncture needle 13 on the basis of the first image acquired by the imaging apparatus that images the puncture needle 13 or the ultrasound probe 12. The probe coordinate acquisition unit 23 acquires the distal end coordinates of the ultrasound probe 12 on the basis of the first image. The target region coordinate acquisition unit 24 acquires the coordinates of the target region in the ultrasound image.

The support information generation unit 25 generates the support information by using at least any one of the distal end coordinates of the puncture needle 13, the distal end coordinates of the ultrasound probe 12, or the coordinates of the target region. The support information is displayed on the display 27 by being displayed on the first image in a superimposed manner. The user performs the operation of the puncture needle 13 while observing the support information displayed on the display 27.

Specifically, in a case where the first image is the rigid endoscope image, the puncture needle 13 is provided with the intracorporeal puncture needle marker, and the ultrasound probe 12 is provided with the intracorporeal puncture needle marker 30, the distal end coordinates of the puncture needle 13 and the distal end coordinates of the ultrasound probe 12 can be obtained from the rigid endoscope image. Then, as the support information, the extension line 42 extending from the distal end of the puncture needle 13 is generated on the basis of the distal end coordinates of the puncture needle 13, and is displayed on the display 27. **In** addition, as the support information, the cross section guide 40 that displays the position of the ultrasound cross section is generated on the basis of the distal end coordinates of the ultrasound probe 12, and is displayed on the display 27.

The user operates the puncture needle 13 in the body while observing the extension line 42 and the cross section guide 40 displayed on the display. The extension line 42 is also moved following the operation of the puncture needle 13. Then, in a case where the puncture needle 13 is moved to an appropriate position, such as a case where the extension line 42 overlaps the target region, the puncture needle 13 punctures the target site.

**In** the embodiment described above, the hardware structures of processing units that execute various kinds of processing, such as the image acquisition unit 21, the puncture needle coordinate acquisition unit 22, the probe coordinate acquisition unit 23, the target region coordinate acquisition unit 24, the support information generation unit 25, and the display controller 26 are various processors as described below. The various processors include a central processing unit (CPU) that is a general-purpose processor which functions as various processing units by executing software (program), a graphical processing unit (GPU), a programmable logic device (PLD) that is a processor capable of changing a circuit configuration after manufacture, such as a field programmable gate array (FPGA), and a dedicated electric circuit that is a processor having a circuit configuration specifically designed to execute various kinds of processing.

One processing unit may be composed of one of the various processors or may be composed of a combination of two or more processors of the same type or different types (for example, a combination of a plurality of FPGAs, a combination of a CPU and an FPGA, or a combination of a CPU and a GPU). In addition, one processor may constitute a plurality of processing units. As an example where a plurality of processing units are configured by one processor, first, there is an aspect where one processor is configured by a combination of one or more CPUs and software as typified by a computer, such as a client or a server, and this processor functions as a plurality of processing units. Second, there is an aspect where a processor fulfilling the functions of the entire system including a plurality of processing units by one integrated circuit (IC) chip as typified by a system on chip (SoC) or the like is used. As described above, the various processing units are configured using one or more of the various processors as a hardware structure.

Furthermore, the hardware structures of the various processors are more specifically electrical circuitry in a form in which circuit elements, such as semiconductor elements, are combined. In addition, a hardware structure of the storage unit is a storage device such as a hard disc drive (HDD) and a solid state drive (SSD).

### Explanation of References

10: laparoscopic system
11: rigid endoscope
12: ultrasound probe
13: puncture needle
14: camera
20: puncture support apparatus
21: image acquisition unit
22: puncture needle coordinate acquisition unit
23: probe coordinate acquisition unit
24: target region coordinate acquisition unit
25: support information generation unit
26: display controller
27: display
28: ultrasound image
29: target region
30: intracorporeal puncture needle marker
31: intracorporeal probe marker
33: extracorporeal puncture needle marker
34: extracorporeal probe marker
36: extracorporeal rigid endoscope marker
40: cross section guide
42: extension line
44: needle insertion hole
46: puncture needle guide
48: puncture point
49: gradation
50: shape model
52: lattice-shaped rectangular parallelepiped

## Claims

1. A puncture support apparatus (20) comprising:
a processor,
wherein the processor is configured to:
acquire a first image acquired by an imaging apparatus that images a puncture needle (13) and an ultrasound probe (12);
acquire distal end coordinates of the puncture needle (13) on the basis of the first image;
acquire distal end coordinates of the ultrasound probe (12) on the basis of the first image;
acquire coordinates of a target region in an ultrasound image acquired by the ultrasound probe (12);
generate support information for supporting a puncture operation of the puncture needle (13), by using at least any one of the distal end coordinates of the puncture needle (13), the distal end coordinates of the ultrasound probe (12), or the coordinates of the target region; and
display the support information on a display (27) by superimposing the support information on the first image;
**characterized in that**
the first image is a rigid endoscope image captured by a rigid endoscope (11) that is the imaging apparatus,
an intracorporeal probe marker (31) is attached to a portion of the ultrasound probe (12), which is inserted into the body, and
the distal end coordinates of the ultrasound probe are calculated on the basis of the intracorporeal probe marker (31) included in the rigid endoscope image.

2. The puncture support apparatus according to claim 1,
wherein an intracorporeal puncture needle marker (30) is attached to a portion of the puncture needle (13), which is inserted into a body,
the distal end coordinates of the puncture needle (13) are calculated on the basis of the intracorporeal puncture needle marker (30) included in the rigid endoscope image,

3. The puncture support apparatus according to claim 1,
wherein the processor is configured to acquire the first image and a second image that is different from the first image, from a plurality of imaging apparatuses,
the second image is a camera image acquired by a camera (14) that is installed outside a body and is the imaging apparatus,
an extracorporeal puncture needle marker (33) is attached to a portion of the puncture needle (13), which is positioned outside the body, and
the distal end coordinates of the puncture needle (13) are calculated on the basis of the extracorporeal puncture needle marker (33) included in the camera image.

4. The puncture support apparatus according to claim 3,
wherein an extracorporeal rigid endoscope marker (36) is attached to a portion of the rigid endoscope (11), which is positioned outside the body, and
distal end coordinates of the rigid endoscope (11) are calculated on the basis of the extracorporeal rigid endoscope marker (36) included in the camera image.

5. The puncture support apparatus according to any one of claims 1 to 4,
wherein the processor is configured to generate a cross section guide that displays a position of an ultrasound cross section, as the support information, on the basis of the distal end coordinates of the ultrasound probe (12).

6. The puncture support apparatus according to any one of claims 1 to 4,
wherein the processor is configured to generate an extension line extending from a distal end of the puncture needle, as the support information, on the basis of the distal end coordinates of the puncture needle (13).

7. The puncture support apparatus according to any one of claims 1 to 4,
wherein the processor is configured to:
generate a cross section guide that displays a position of an ultrasound cross section, as the support information, on the basis of the distal end coordinates of the ultrasound probe (12); and
make a display aspect of the support information different, between a case where the support information is on a front side of at least any one of the cross section guide, the ultrasound probe, or a cross section of the ultrasound image, and a case where the support information overlaps or is on a back side of at least any one of the cross section guide, the ultrasound probe, or the cross section of the ultrasound image.

8. The puncture support apparatus according to any one of claims 1 to 4,
wherein the processor is configured to:
generate a cross section guide that displays a position of an ultrasound cross section, as the support information, on the basis of the distal end coordinates of the ultrasound probe (12);
generate an extension line extending from a distal end of the puncture needle (13), as the support information, on the basis of the distal end coordinates of the puncture needle (13); and
make a display aspect of the extension line different, between a case where the extension line is on a front side of at least any one of the cross section guide, the ultrasound probe (12), or a cross section of the ultrasound image, and a case where the extension line overlaps or is on a back side of at least any one of the cross section guide, the ultrasound probe (12), or the cross section of the ultrasound image.

9. The puncture support apparatus according to any one of claims 1 to 4,
wherein the processor is configured to generate a puncture needle guide (46) for guiding the puncture needle (13), in accordance with a position of a needle insertion hole (41) of the ultrasound probe (13), as the support information, on the basis of the distal end coordinates of the ultrasound probe (12), and display the puncture needle guide (46) on the first image in a superimposed manner.

10. The puncture support apparatus according to any one of claims 1 to 4,
wherein the processor is configured to generate a puncture point (48) that is a target insertion position of the puncture needle (13), as the support information, on the basis of the coordinates of the target region.

11. The puncture support apparatus according to any one of claims 1 to 4,
wherein the processor is configured to:
generate an extension line (42) extending from a distal end of the puncture needle (13), as the support information, on the basis of the distal end coordinates of the puncture needle (13);
generate a puncture point (48) that is a target insertion position of the puncture needle (13), as the support information, on the basis of the coordinates of the target region; and
generate a distance from the distal end of the puncture needle (13) to the target region, as the support information in a case where the extension line (42) overlaps the puncture point (48).

12. The puncture support apparatus according to any one of claims 1 to 4,
wherein the processor is configured to:
generate a three-dimensional shape model of the target region, as the support information, on the basis of the ultrasound image and the coordinates of the target region; and
generate a three-dimensional relationship from a distal end of the puncture needle (13) to the target region, as the support information, on the basis of the distal end coordinates of the puncture needle (13) and the coordinates of the target region.

13. An operation method of a puncture support apparatus including a processor, the operation method comprising:
via the processor,
acquiring a first image acquired by an imaging apparatus that images a puncture needle (12) and an ultrasound probe (13);
acquiring distal end coordinates of the puncture needle (13) on the basis of the first image;
acquiring distal end coordinates of the ultrasound probe (12) on the basis of the first image;
acquiring coordinates of a target region in an ultrasound image acquired by the ultrasound probe (12);
generating support information for supporting a puncture operation of the puncture needle (13) by using at least any one of the distal end coordinates of the puncture needle (13), the distal end coordinates of the ultrasound probe (12), or the coordinates of the target region; and
displaying the support information on a display (27) by superimposing the support information on the first image;
**characterized in that**
the first image is a rigid endoscope image captured by a rigid endoscope (11) that is the imaging apparatus,
an intracorporeal probe marker (31) is attached to a portion of the ultrasound probe (12), which is inserted into the body, and
the distal end coordinates of the ultrasound probe (12) are calculated on the basis of the intracorporeal probe marker (31) included in the rigid endoscope image.

14. A non-transitory computer readable medium for storing a computer-executable program, the computer-executable program causing a processor to execute:
a function of acquiring a first image acquired by an imaging apparatus that images a puncture needle (13) and an ultrasound probe (12);
a function of acquiring distal end coordinates of the puncture needle (13) on the basis of the first image;
a function of acquiring distal end coordinates of the ultrasound probe (12) on the basis of the first image;
a function of acquiring coordinates of a target region in an ultrasound image acquired by the ultrasound probe (12);
a function of generating support information for supporting a puncture operation of the puncture needle (13) by using at least any one of the distal end coordinates of the puncture needle (13), the distal end coordinates of the ultrasound probe (12), or the coordinates of the target region; and
a function of displaying the support information on a display (27) by superimposing the support information on the first image;
**characterized in that**
the first image is a rigid endoscope image captured by a rigid endoscope (11) that is the imaging apparatus,
an intracorporeal probe marker (31) is attached to a portion of the ultrasound probe (12), which is inserted into the body, and
the distal end coordinates of the ultrasound probe are calculated on the basis of the intracorporeal probe marker (31) included in the rigid endoscope image.

## Patentansprüche

1. Punktionsunterstützungsvorrichtung (20), umfassend:
einen Prozessor,
wobei der Prozessor so konfiguriert ist, dass er:
ein erstes Bild, das von einer Bildgebungsvorrichtung, die eine Punktionskanüle (13) und eine Ultraschallsonde (12) abbildet, erfasst wird, erfasst;
distale Endkoordinaten der Punktionskanüle (13) auf der Grundlage des ersten Bildes erfasst;
distale Endkoordinaten der Ultraschallsonde (12) auf der Grundlage des ersten Bildes erfasst;
Koordinaten eines Zielbereichs in einem Ultraschallbild, das von der Ultraschallsonde (12) erfasst wird, erfasst;
Unterstützungsinformationen zum Unterstützen eines Punktionsvorgangs der Punktionskanüle (13) durch Verwenden von mindestens eins von den distalen Endkoordinaten der Punktionskanüle (13), den distalen Endkoordinaten der Ultraschallsonde (12) und den Koordinaten des Zielbereichs erzeugt; und
die Unterstützungsinformationen auf einer Anzeige (27) anzeigt, indem er die Unterstützungsinformationen dem ersten Bild überlagert;
**dadurch gekennzeichnet, dass**
das erste Bild ein starres Endoskopbild ist, das von einem starren Endoskop (11) aufgenommen worden ist, das die Bildgebungsvorrichtung ist,
eine intrakorporale Sondenmarkierung (31) an einem Abschnitt der Ultraschallsonde (12), der in den Körper eingeführt wird, angebracht ist, und
die distalen Endkoordinaten der Ultraschallsonde auf der Grundlage der intrakorporalen Sondenmarkierung (31), die in dem starren Endoskopbild enthalten ist, berechnet werden.

2. Punktionsunterstützungsvorrichtung nach Anspruch 1,
wobei eine intrakorporale Punktionskanülenmarkierung (30) an einem Abschnitt der Punktionskanüle (13), der in einen Körper eingeführt wird, angebracht ist, und
die distalen Endkoordinaten der Punktionskanüle (13) auf der Grundlage der intrakorporalen Punktionskanülenmarkierung (30), die in dem starren Endoskopbild enthalten ist, berechnet werden.

3. Punktionsunterstützungsvorrichtung nach Anspruch 1,
wobei der Prozessor so konfiguriert ist, dass er das erste Bild und ein zweites Bild, das sich von dem ersten Bild unterscheidet, von mehreren Bildgebungsvorrichtungen erfasst,
das zweite Bild ein Kamerabild ist, das von einer Kamera (14) erfasst wird, die außerhalb eines Körpers installiert ist und die Bildgebungsvorrichtung ist,
eine extrakorporale Punktionskanülenmarkierung (33) an einem Abschnitt der Punktionskanüle (13), der außerhalb des Körpers positioniert ist, angebracht ist, und die distalen Endkoordinaten der Punktionskanüle (13) auf der Grundlage der extrakorporalen Punktionskanülenmarkierung (33), die in dem Kamerabild enthalten ist, berechnet werden.

4. Punktionsunterstützungsvorrichtung nach Anspruch 3,
wobei eine extrakorporale starre Endoskopmarkierung (36) an einem Abschnitt des starren Endoskops (11), der außerhalb des Körpers positioniert ist, angebracht ist, und
distale Endkoordinaten des starren Endoskops (11) auf der Grundlage der extrakorporalen starren Endoskopmarkierung (36), die in dem Kamerabild enthalten ist, berechnet werden.

5. Punktionsunterstützungsvorrichtung nach einem der Ansprüche 1 bis 4,
wobei der Prozessor so konfiguriert ist, dass er eine Querschnittsführung, die eine Position eines Ultraschallquerschnitts anzeigt, als die Unterstützungsinformationen auf der Grundlage der distalen Endkoordinaten der Ultraschallsonde (12) erzeugt.

6. Punktionsunterstützungsvorrichtung nach einem der Ansprüche 1 bis 4,
wobei der Prozessor so konfiguriert ist, dass er eine Verlängerungslinie, die sich von einem distalen Ende der Punktionskanüle erstreckt, als die Unterstützungsinformationen auf der Grundlage der distalen Endkoordinaten der Punktionskanüle (13) erzeugt.

7. Punktionsunterstützungsvorrichtung nach einem der Ansprüche 1 bis 4,
wobei der Prozessor so konfiguriert ist, dass er:
eine Querschnittsführung, die eine Position eines Ultraschallquerschnitts anzeigt, als die Unterstützungsinformationen auf der Grundlage der distalen Endkoordinaten der Ultraschallsonde (12) erzeugt; und
eine Anzeigeansicht der Unterstützungsinformationen zwischen einem Fall, in dem sich die Unterstützungsinformationen auf einer Vorderseite von mindestens einem von der Querschnittsführung, der Ultraschallsonde und einem Querschnitt des Ultraschallbildes befinden, und einem Fall, in dem sich die Unterstützungsinformationen mit mindestens einem von der Querschnittsführung, der Ultraschallsonde und dem Querschnitt des Ultraschallbildes überlappen oder sich auf einer Rückseite davon befinden, unterschiedlich macht.

8. Punktionsunterstützungsvorrichtung nach einem der Ansprüche 1 bis 4,
wobei der Prozessor so konfiguriert ist, dass er:
eine Querschnittsführung, die eine Position eines Ultraschallquerschnitts anzeigt, als die Unterstützungsinformationen auf der Grundlage der distalen Endkoordinaten der Ultraschallsonde (12) erzeugt;
eine Verlängerungslinie, die sich von einem distalen Ende der Punktionskanüle (13) erstreckt, als die Unterstützungsinformationen auf der Grundlage der distalen Endkoordinaten der Punktionskanüle (13) erzeugt; und
eine Anzeigeansicht der Verlängerungslinie zwischen einem Fall, in dem sich die Verlängerungslinie auf einer Vorderseite von mindestens einem von der Querschnittsführung, der Ultraschallsonde (12) und einem Querschnitt des Ultraschallbildes befindet, und einem Fall, in dem sich die Verlängerungslinie mit mindestens einem von der Querschnittsführung, der Ultraschallsonde (12) und dem Querschnitt des Ultraschallbildes überlappen oder sich auf einer Rückseite davon befindet, unterschiedlich macht.

9. Punktionsunterstützungsvorrichtung nach einem der Ansprüche 1 bis 4,
wobei der Prozessor so konfiguriert ist, dass er eine Punktionskanülenführung (46) zum Führen der Punktionskanüle (13) in Übereinstimmung mit einer Position eines Kanüleneinführlochs (41) der Ultraschallsonde (13) als die Unterstützungsinformationen auf der Grundlage der distalen Endkoordinaten der Ultraschallsonde (12) erzeugt und die Punktionskanülenführung (46) auf dem ersten Bild überlagert anzeigt.

10. Punktionsunterstützungsvorrichtung nach einem der Ansprüche 1 bis 4,
wobei der Prozessor so konfiguriert ist, dass er einen Punktionspunkt (48), der eine Zieleinführposition der Punktionskanüle (13) ist, als die Unterstützungsinformationen auf der Grundlage der Koordinaten des Zielbereichs erzeugt.

11. Punktionsunterstützungsvorrichtung nach einem der Ansprüche 1 bis 4, wobei der Prozessor so konfiguriert ist, dass er:
eine Verlängerungslinie (42), die sich von einem distalen Ende der Punktionskanüle (13) erstreckt, als die Unterstützungsinformationen auf der Grundlage der distalen Endkoordinaten der Punktionskanüle (13) erzeugt;
einen Punktionspunkt (48), der eine Zieleinführposition der Punktionskanüle (13) ist, als die Unterstützungsinformationen auf der Grundlage der Koordinaten des Zielbereichs erzeugt; und
eine Entfernung von dem distalen Ende der Punktionskanüle (13) zu dem Zielbereich als die Unterstützungsinformationen in einem Fall, in dem die Verlängerungslinie (42) den Punktionspunkt (48) überlappt, erzeugt.

12. Punktionsunterstützungsvorrichtung nach einem der Ansprüche 1 bis 4,
wobei der Prozessor so konfiguriert ist, dass er:
ein dreidimensionales Formmodell des Zielbereichs als die Unterstützungsinformationen auf der Grundlage des Ultraschallbildes und der Koordinaten des Zielbereichs erzeugt; und
eine dreidimensionale Beziehung von einem distalen Ende der Punktionskanüle (13) zu dem Zielbereich als die Unterstützungsinformationen auf der Grundlage der distalen Endkoordinaten der Punktionskanüle (13) und der Koordinaten des Zielbereichs erzeugt.

13. Betriebsverfahren einer Punktionsunterstützungsvorrichtung, die einen Prozessor enthält, wobei das Betriebsverfahren umfasst:
via den Prozessor,
Erfassen eines ersten Bildes, das von einer Bildgebungsvorrichtung, die eine Punktionskanüle (12) und eine Ultraschallsonde (13) abbildet, erfasst wird;
Erfassen von distalen Endkoordinaten der Punktionskanüle (13) auf der Grundlage des ersten Bildes;
Erfassen von distalen Endkoordinaten der Ultraschallsonde (12) auf der Grundlage des ersten Bildes;
Erfassen von Koordinaten eines Zielbereichs in einem Ultraschallbild, das von der Ultraschallsonde (12) erfasst wird;
Erzeugen von Unterstützungsinformationen zum Unterstützen eines Punktionsvorgangs der Punktionskanüle (13) durch Verwenden von mindestens eins von den distalen Endkoordinaten der Punktionskanüle (13), den distalen Endkoordinaten der Ultraschallsonde (12) und den Koordinaten des Zielbereichs; und
Anzeigen der Unterstützungsinformationen auf einer Anzeige (27), indem die Unterstützungsinformationen dem ersten Bild überlagert werden;
**dadurch gekennzeichnet, dass**
das erste Bild ein starres Endoskopbild ist, das von einem starren Endoskop (11) aufgenommen worden ist, das die Bildgebungsvorrichtung ist,
eine intrakorporale Sondenmarkierung (31) an einem Abschnitt der Ultraschallsonde (12), der in den Körper eingeführt wird, angebracht ist, und
die distalen Endkoordinaten der Ultraschallsonde (12) auf der Grundlage der intrakorporalen Sondenmarkierung (31), die in dem starren Endoskopbild enthalten ist, berechnet werden.

14. Nicht flüchtiges computerlesbares Medium zum Speichern eines computerausführbaren Programms, wobei das computerausführbare Programm einen Prozessor veranlasst, auszuführen:
eine Funktion des Erfassens eines ersten Bildes, das von einer
Bildgebungsvorrichtung, die eine Punktionskanüle (13) und eine Ultraschallsonde (12) abbildet, erfasst wird;
eine Funktion des Erfassens von distalen Endkoordinaten der Punktionskanüle (13) auf der Grundlage des ersten Bildes;
eine Funktion des Erfassens von distalen Endkoordinaten der Ultraschallsonde (12) auf der Grundlage des ersten Bildes;
eine Funktion des Erfassens von Koordinaten eines Zielbereichs in einem Ultraschallbild, das von der Ultraschallsonde (12) erfasst wird;
eine Funktion des Erzeugens von Unterstützungsinformationen zum Unterstützen eines Punktionsvorgangs der Punktionskanüle (13) durch Verwenden von mindestens eins von den distalen Endkoordinaten der Punktionskanüle (13), den distalen Endkoordinaten der Ultraschallsonde (12) und den Koordinaten des Zielbereichs; und
eine Funktion des Anzeigens der Unterstützungsinformationen auf einer Anzeige (27), indem die Unterstützungsinformationen dem ersten Bild überlagert werden;
**dadurch gekennzeichnet, dass**
das erste Bild ein starres Endoskopbild ist, das von einem starren Endoskop (11) aufgenommen worden ist, das die Bildgebungsvorrichtung ist,
eine intrakorporale Sondenmarkierung (31) an einem Abschnitt der Ultraschallsonde (12), der in den Körper eingeführt wird, angebracht ist, und
die distalen Endkoordinaten der Ultraschallsonde auf der Grundlage der intrakorporalen Sondenmarkierung (31), die in dem starren Endoskopbild enthalten ist, berechnet werden.

## Revendications

1. Appareil d'assistance à la ponction (20) comprenant :
un processeur,
dans lequel le processeur est configuré pour :
acquérir une première image acquise par un appareil d'imagerie qui image une aiguille de ponction (13) et une sonde ultrasonore (12) ;
acquérir des coordonnées d'extrémité distale de l'aiguille de ponction (13) sur la base de la première image ;
acquérir des coordonnées d'extrémité distale de la sonde ultrasonore (12) sur la base de la première image ;
acquérir des coordonnées d'une région cible dans une image ultrasonore acquise par la sonde ultrasonore (12) ;
générer des informations d'assistance pour assister une opération de ponction de l'aiguille de ponction (13), en utilisant au moins l'une quelconque des coordonnées d'extrémité distale de l'aiguille de ponction (13), des coordonnées d'extrémité distale de la sonde ultrasonore (12) ou des coordonnées de la région cible ; et
afficher les informations d'assistance sur un affichage (27) en superposant les informations d'assistance sur la première image ;
**caractérisé en ce que**
la première image est une image d'endoscope rigide capturée par un endoscope rigide (11) qui est l'appareil d'imagerie,
un marqueur de sonde intracorporelle (31) est fixé à une partie de la sonde ultrasonore (12), qui est insérée dans le corps, et
les coordonnées d'extrémité distale de la sonde ultrasonore sont calculées sur la base du marqueur de sonde intracorporelle (31) inclus dans l'image d'endoscope rigide.

2. Appareil d'assistance à la ponction selon la revendication 1,
dans lequel un marqueur intracorporel d'aiguille de ponction (30) est fixé à une partie de l'aiguille de ponction (13), qui est insérée dans un corps, et
les coordonnées d'extrémité distale de l'aiguille de ponction (13) sont calculées sur la base du marqueur intracorporel d'aiguille de ponction (30) inclus dans l'image d'endoscope rigide.

3. Appareil d'assistance à la ponction selon la revendication 1,
dans lequel le processeur est configuré pour acquérir la première image et une deuxième image qui est différente de la première image, à partir d'une pluralité d'appareils d'imagerie,
la deuxième image est une image de caméra acquise par une caméra (14) qui est installée à l'extérieur d'un corps et est l'appareil d'imagerie,
un marqueur d'aiguille de ponction extracorporelle (33) est fixé à une partie de l'aiguille de ponction (13), qui est positionnée à l'extérieur du corps, et
les coordonnées d'extrémité distale de l'aiguille de ponction (13) sont calculées sur la base du marqueur d'aiguille de ponction extracorporelle (33) inclus dans l'image de caméra.

4. Appareil d'assistance à la ponction selon la revendication 3,
dans lequel un marqueur d'endoscope rigide extracorporel (36) est fixé à une partie de l'endoscope rigide (11), qui est positionné à l'extérieur du corps, et
des coordonnées d'extrémité distale de l'endoscope rigide (11) sont calculées sur la base du marqueur d'endoscope rigide extracorporel (36) inclus dans l'image de caméra.

5. Appareil d'assistance à la ponction selon l'une quelconque des revendications 1 à 4,
dans lequel le processeur est configuré pour générer un guide de section transversale qui affiche une position d'une section transversale ultrasonore, comme informations d'assistance, sur la base des coordonnées d'extrémité distale de la sonde ultrasonore (12).

6. Appareil d'assistance à la ponction selon l'une quelconque des revendications 1 à 4,
dans lequel le processeur est configuré pour générer une ligne d'extension s'étendant à partir d'une extrémité distale de l'aiguille de ponction, comme informations d'assistance, sur la base des coordonnées d'extrémité distale de l'aiguille de ponction (13).

7. Appareil d'assistance à la ponction selon l'une quelconque des revendications 1 à 4,
dans lequel le processeur est configuré pour :
générer un guide de section transversale qui affiche une position d'une section transversale ultrasonore, comme informations d'assistance, sur la base des coordonnées d'extrémité distale de la sonde ultrasonore (12) ; et
rendre différent un aspect d'affichage des informations d'assistance, entre un cas où les informations d'assistance sont situées sur un côté avant d'au moins l'un quelconque du guide de section transversale, de la sonde ultrasonore ou d'une section transversale de l'image ultrasonore, et un cas où les informations d'assistance chevauchent ou sont situées sur un côté arrière d'au moins l'un quelconque du guide de section transversale, de la sonde ultrasonore ou de la section transversale de l'image ultrasonore.

8. Appareil d'assistance à la ponction selon l'une quelconque des revendications 1 à 4,
dans lequel le processeur est configuré pour :
générer un guide de section transversale qui affiche une position d'une section transversale ultrasonore, comme informations d'assistance, sur la base des coordonnées d'extrémité distale de la sonde ultrasonore (12) ;
générer une ligne d'extension s'étendant à partir d'une extrémité distale de l'aiguille de ponction (13), comme informations d'assistance, sur la base des coordonnées d'extrémité distale de l'aiguille de ponction (13) ; et
rendre différent un aspect d'affichage de la ligne d'extension, entre un cas où la ligne d'extension est située sur un côté avant d'au moins l'un quelconque du guide de section transversale, de la sonde ultrasonore (12) ou d'une section transversale de l'image ultrasonore, et un cas où la ligne d'extension chevauche ou est située sur un côté arrière d'au moins l'un quelconque du guide de section transversale, de la sonde ultrasonore (12) ou de la section transversale de l'image ultrasonore.

9. Appareil d'assistance à la ponction selon l'une quelconque des revendications 1 à 4,
dans lequel le processeur est configuré pour générer un guide d'aiguille de ponction (46) pour guider l'aiguille de ponction (13), conformément à une position d'un orifice d'insertion d'aiguille (41) de la sonde ultrasonore (13), comme informations d'assistance, sur la base des coordonnées d'extrémité distale de la sonde ultrasonore (12), et pour afficher le guide d'aiguille de ponction (46) sur la première image de manière superposée.

10. Appareil d'assistance à la ponction selon l'une quelconque des revendications 1 à 4,
dans lequel le processeur est configuré pour générer un point de ponction (48) qui est une position d'insertion cible de l'aiguille de ponction (13), comme informations d'assistance, sur la base des coordonnées de la région cible.

11. Appareil d'assistance à la ponction selon l'une quelconque des revendications 1 à 4,
dans lequel le processeur est configuré pour :
générer une ligne d'extension (42) s'étendant à partir d'une extrémité distale de l'aiguille de ponction (13), comme informations d'assistance, sur la base des coordonnées d'extrémité distale de l'aiguille de ponction (13) ;
générer un point de ponction (48) qui est une position d'insertion cible de l'aiguille de ponction (13), comme informations d'assistance, sur la base des coordonnées de la région cible ; et
générer une distance entre l'extrémité distale de l'aiguille de ponction (13) et la région cible, comme informations d'assistance dans un cas où la ligne d'extension (42) chevauche le point de ponction (48).

12. Appareil d'assistance à la ponction selon l'une quelconque des revendications 1 à 4,
dans lequel le processeur est configuré pour :
générer un modèle de forme tridimensionnel de la région cible, comme informations d'assistance, sur la base de l'image ultrasonore et des coordonnées de la région cible ; et
générer une relation tridimensionnelle entre une extrémité distale de l'aiguille de ponction (13) et la région cible, comme informations d'assistance, sur la base des coordonnées d'extrémité distale de l'aiguille de ponction (13) et des coordonnées de la région cible.

13. Procédé d'opération d'un appareil d'assistance à la ponction incluant un processeur, le procédé d'opération comprenant :
via le processeur,
acquérir une première image acquise par un appareil d'imagerie qui image une aiguille de ponction (12) et une sonde ultrasonore (13) ;
acquérir des coordonnées d'extrémité distale de l'aiguille de ponction (13) sur la base de la première image ;
acquérir des coordonnées d'extrémité distale de la sonde ultrasonore (12) sur la base de la première image ;
acquérir des coordonnées d'une région cible dans une image ultrasonore acquise par la sonde ultrasonore (12) ;
générer des informations d'assistance pour assister une opération de ponction de l'aiguille de ponction (13), en utilisant au moins l'une quelconque des coordonnées d'extrémité distale de l'aiguille de ponction (13), des coordonnées d'extrémité distale de la sonde ultrasonore (12) ou des coordonnées de la région cible ; et
afficher les informations d'assistance sur un affichage (27) en superposant les informations d'assistance sur la première image ;
**caractérisé en ce que**
la première image est une image d'endoscope rigide capturée par un endoscope rigide (11) qui est l'appareil d'imagerie,
un marqueur de sonde intracorporelle (31) est fixé à une partie de la sonde ultrasonore (12), qui est insérée dans le corps, et
les coordonnées d'extrémité distale de la sonde ultrasonore (12) sont calculées sur la base du marqueur de sonde intracorporelle (31) inclus dans l'image d'endoscope rigide.

14. Support non transitoire lisible par ordinateur pour stocker un programme exécutable par ordinateur, le programme exécutable par ordinateur amenant un processeur à exécuter :
une fonction consistant à acquérir une première image acquise par un appareil d'imagerie qui image une aiguille de ponction (13) et une sonde ultrasonore (12) ;
une fonction consistant à acquérir des coordonnées d'extrémité distale de l'aiguille de ponction (13) sur la base de la première image ;
une fonction consistant à acquérir des coordonnées d'extrémité distale de la sonde ultrasonore (12) sur la base de la première image ;
une fonction consistant à acquérir des coordonnées d'une région cible dans une image ultrasonore acquise par la sonde ultrasonore (12) ;
une fonction consistant à générer des informations d'assistance pour assister une opération de ponction de l'aiguille de ponction (13), en utilisant au moins l'une quelconque des coordonnées d'extrémité distale de l'aiguille de ponction (13), des coordonnées d'extrémité distale de la sonde ultrasonore (12) ou des coordonnées de la région cible ; et
une fonction consistant à afficher des informations d'assistance sur un affichage (27) en superposant les informations d'assistance sur la première image ;
**caractérisé en ce que**
la première image est une image d'endoscope rigide capturée par un endoscope rigide (11) qui est l'appareil d'imagerie,
un marqueur de sonde intracorporelle (31) est fixé à une partie de la sonde ultrasonore (12), qui est insérée dans le corps, et
les coordonnées d'extrémité distale de la sonde ultrasonore sont calculées sur la base du marqueur de sonde intracorporelle (31) inclus dans l'image d'endoscope rigide.
